# EUROPÄISCHE PATENTANMELDUNG

(11) **EP 4 205 850 A1**
(43) Veröffentlichungstag der Anmeldung: **05.07.2023**
(21) Anmeldenummer: 22150078.8
(22) Anmeldetag: 03.01.2022
(51) Int. Cl.: B01L 3/00, A61B 10/00

(54) **VORRICHTUNG ZUR AUFNAHME VON PROBEN**

(71) Anmelder: Procomcure Biotech GmbH, 5303 Thalgau (AT)
(72) Erfinder: ÖNDER, Kamil, 5301 Eugendorf (AT)
(74) Vertreter: dompatent von Kreisler Selting Werner - Partnerschaft von Patent- und Rechtsanwälten mbB

(57) **Zusammenfassung**

Bei einer Vorrichtung (1) zur Aufnahme von Proben, insbesondere Speichelproben zum Nachweis von SARS-CoV-2 Virus im Speichel, mit
- einem Aufnahmebehälter (2) mit einer ersten Öffnung (4),
- einer Verschlusseinrichtung (6), die an die Öffnung (4) des Aufnahmebehälters (2) angepasst ist, so dass die Öffnung (4) des Aufnahmebehälters (2) mit der Verschlusseinrichtung (6) verschließbar ist,
- einer Probenentnahmeeinrichtung (8), die an einem ersten freien Ende (10) zumindest einen ersten Entnahmeelement (12) aufweist, mit dem die Probe entnehmbar ist, und wobei die Probenentnahmeeinrichtung (8) in den Aufnahmebehälter (2) einführbar ist, kann vorgesehen, dass die Probenentnahmeeinrichtung (8) an einem dem ersten freien Ende (10) gegenüberliegenden zweiten Ende (14) mit der Verschlusseinrichtung (6) verbunden ist, so dass die Probenentnahmeeinrichtung (8) mittels der Verschlusseinrichtung (6) in den Aufnahmebehälter (2) einführbar ist.

## Beschreibung

Die Erfindung betrifft eine Vorrichtung zur Aufnahme von Proben, insbesondere Speichelproben zum Nachweis von SARS-CoV-2 Virus im Speichel nach dem Oberbegriff des Anspruchs 1, sowie ein Verfahren zur Aufnahme von Proben, insbesondere Speichelproben zum Nachweis von SARS-CoV-2 Virus im Speichel nach dem Oberbegriff des Anspruchs 10.

Es sind Vorrichtungen zur Aufnahme von Proben, insbesondere Speichelproben zum Nachweis von SARS-CoV-2 Virus im Speichel bekannt, mit einem Aufnahmebehälter mit einer ersten Öffnung, einer Verschlusseinrichtung, die an die Öffnung des Aufnahmebehälters angepasst ist, so dass die Öffnung des Aufnahmebehälters mit der Verschlusseinrichtung verschließbar ist, einer Probenentnahmeeinrichtung, die an einem ersten freien Ende zumindest einen ersten Entnahmeelement aufweist, mit dem die Probe entnehmbar ist, und wobei die Probenentnahmeeinrichtung in den Aufnahmebehälter einführbar ist,

Es besteht jedoch zunehmen Bedarf, diese Vorrichtungen zu vereinfachen.

Die Aufgabe der vorliegenden Erfindung ist es daher eine Vorrichtung und ein Verfahren zu schaffen, die bzw. das einfach zu verwenden ist.

Zur Lösung dieser Aufgabe dienen die Merkmale der Ansprüche 1 und 10.

Die Erfindung sieht in vorteilhafter Weise vor, dass die Probenentnahmeeinrichtung an einem dem ersten freien Ende gegenüberliegenden zweiten Ende mit der Verschlusseinrichtung verbunden ist, sodass die Probenentnahmeeinrichtung mittels der Verschlusseinrichtung in den Aufnahmebehälter einführbar ist.

Die vorliegende Erfindung hat den Vorteil, dass die Entnahme der Proben vereinfacht ist. Es muss nicht zunächst die Probenentnahmeeinrichtung woanders platziert werden, um die Verschlusseinrichtung zu öffnen. Und andererseits muss die Verschlusseinrichtung auch nirgendwo platziert werden, damit die Probenentnahmeeinrichtung in den Aufnahmebehälter eingeführt werden kann.

Die Probenentnahmeeinrichtung kann zumindest ein hohles stabförmiges Element aufweisen, an dessen freien Ende das Entnahmeelement angeordnet ist, wobei das hohle stabförmige Element im Inneren einen Kanal bildet.

Das Entnahmeelement kann aus einem Material bestehen, das bezogen auf Flüssigkeiten saug- oder haftfähig sein kann. Das heißt die das Entnahmeelement kann die Flüssigkeit aufsaugen oder die Flüssigkeit haftet an dem Entnahmeelement.

Ein saugfähiges Material kann beispielsweise ein aus Watte bestehendes Element sein. Alternativ kann es jegliches andere Element sein, dass Feuchtigkeit aufnehmen kann.

Die Aufnahmebehälter kann wasserdicht und luftdicht sein.

Die Verschlusseinrichtung kann eine Durchgangsöffnung aufweisen.

Das stabförmige Element kann derart mit der Verschlusseinrichtung verbunden sein, dass der Kanal des stabförmigen Elements in der Durchgangsöffnung mündet oder in dieser angeordnet ist.

Die Durchgangsöffnung des Verschlusselements kann an der dem Probenentnahmeelement gegenüberliegenden Seite mittels eines Abschlusselements verschlossen sein, das mit dem Verschlusselement trennbar befestigt ist.

Nach abgetrennten Abschlusselement bildet die Durchgangsöffnung eine Öffnung nach außen. Mit abgetrenntem Abschlusselement kann in dem Aufnahmebehälter befindliche Flüssigkeit aufgrund von Kapillarwirkung und/oder Druck und/oder Unterdruck aus über den Kanal des stabförmigen Elements und die Durchgangsöffnung nach außen austreten.

Das Abschlusselement kann von dem Verschlusselement durch Abknicken trennbar sein. Alternativ kann das Abschlusselement auch durch Abschrauben von dem Verschlusselement trennbar sein.

Das hohle stabförmige Element kann an dem freien Ende zumindest eine Öffnung aufweisen.

Das Ende des in dem stabförmigen Element angeordneten Kanals kann die Öffnung an dem freien Ende bildet.

Die an dem freien Ende angeordnete Öffnung kann in der Wand des stabförmigen Elements angeordnet sein.

Ferner kann ein Verfahren zur Aufnahme von Proben, insbesondere Speichelproben zum Nachweis von SARS-CoV-2 Virus im Speichel vorgesehen sein, wobei das Verfahren die folgenden Schritte umfasst
- Bereitstellen eines Aufnahmebehälters mit einer ersten Öffnung,
- Entnahme einer Probe mit zumindest einem an einem freien Ende einer Probenentnahmeeinrichtung angeordneten Entnahmeelements, wobei
- die Probenentnahmeeinrichtung mit einem dem ersten freien Ende gegenüberliegenden zweiten Ende mit einer Verschlusseinrichtung verbunden ist und mittels der Verschlusseinrichtung in den Aufnahmebehälter eingeführt wird und der Aufnahmebehälter mit der Verschlusseinrichtung verschlossen wird.

Die Flüssigkeit kann in den Aufnahmebehälter eingeführt werden und das Entnahmeelement der Probenentnahmeeinrichtung kann beim Einführen in den Aufnahmebehälter in die Flüssigkeit eingetaucht werden.

Die Flüssigkeit kann durch einen Kanal, der in einem stabförmigen Element der Probenentnahmeeinrichtung angeordnet ist, zu einer Durchgangsöffnung der Verschlusseinrichtung geführt werden.

Die Flüssigkeit kann aufgrund von Kapillarwirkung und/oder Druck und/oder Unterdruck zu der Durchgangsöffnung geführt werden.

Die Durchgangsöffnung kann mit einem Abschlusselement verschlossen sein und das Abschlusselement kann von der Verschlusseinrichtung abgetrennt werden.

Die Flüssigkeit, die durch den in dem stabförmigen Element der Probenentnahmeeinrichtung angeordneten Kanal zu der Durchgangsöffnung der Verschlusseinrichtung geführt wird, kann aus der Durchgangsöffnung ausgegeben bzw. ausgeschüttet werden, nachdem die Abschlusselement von der Verschlusseinrichtung abgetrennt worden ist.

Im Folgenden wird unter Bezugnahme auf die Zeichnungen Ausführungsbeispiele der vorliegenden Erfindung näher erläutert.

Es zeigen schematisch:
- Fig. 1: eine Vorrichtung zur Aufnahme von Proben gemäß der vorliegenden Erfindung,
- Fig. 2: die Aufnahme von Proben mittels der erfindungsgemäßen Vorrichtung,
- Fig. 3: die erfindungsgemäße Vorrichtung mit verschlossener Verschlusseinrichtung,
- Fig. 4: Vorrichtung aus Fig. 3 mit abgetrenntem Abschlusselement, und

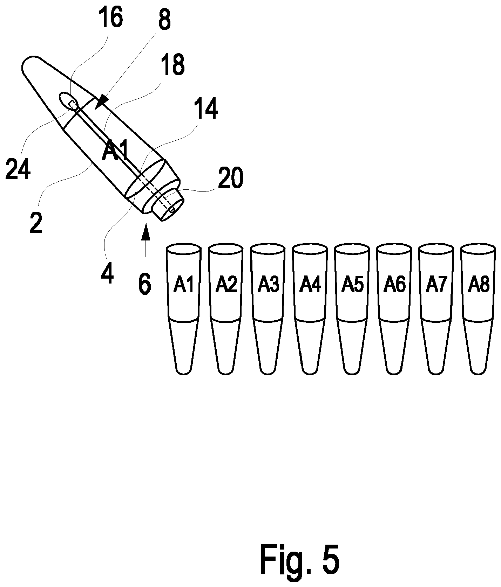

- Fig. 5: Vorrichtung gemäß Fig. 4 bei der Flüssigkeit ausgeschüttet wird.

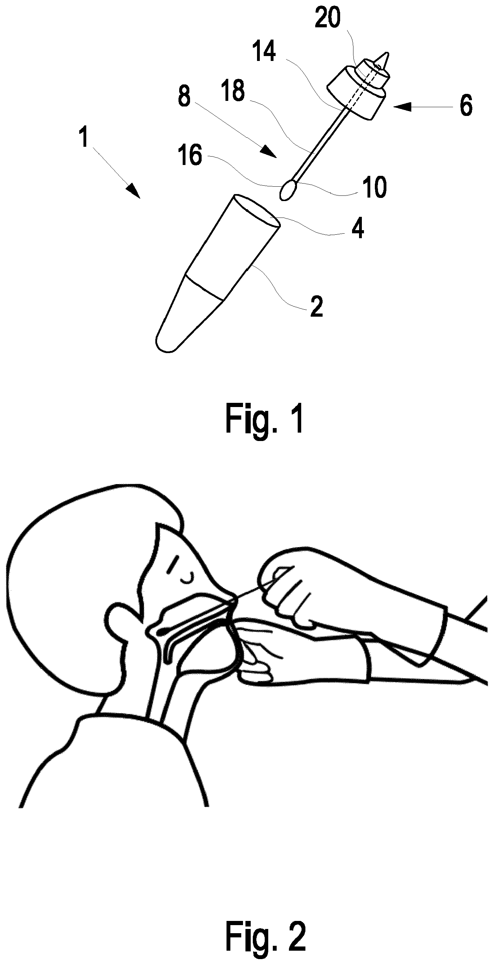

Fig. 1 zeigt die erfindungsgemäße Vorrichtung zur Aufnahme von Proben, insbesondere Speichelproben zum Nachweis von SARS-CoV-2 Virus im Speichel. Die Vorrichtung weist ein Aufnahmebehälter 2 auf, der eine erste Öffnung 4 aufweist.

Ferner ist eine Verschlusseinrichtung 6 vorgesehen, die an die Öffnung 4 des Aufnahmebehälters 2 angepasst ist, so dass die Öffnung 4 des Aufnahmebehälters 2 mit der Verschlusseinrichtung 6 verschlossen werden kann. Ferner ist eine Probenentnahmeeinrichtung 8 dargestellt, die an einem ersten freien Ende 10 zumindest ein erstes Entnahmeelement 12 aufweist. Mit dem Entnahmeelement 12 kann die Probe entnommen werden. Das Entnahmeelement 12 kann aus einem Material bestehen, das bezogen auf Flüssigkeiten saug- oder haftfähig sein kann. Das heißt, das Entnahmeelement kann die Flüssigkeit aufsaugen oder die Flüssigkeit haftet an dem Entnahmeelement.

Ein saugfähiges Material kann beispielsweise ein aus Watte bestehendes Element sein.

Die Probenentnahmeeinrichtung 8 ist an dem ersten freien Ende 10 gegenüberliegenden zweiten Ende 14 mit der Verschlusseinrichtung 6 verbunden. Die Probenentnahmeeinrichtung 8 kann mittels der Verschlusseinrichtung 6 in den Aufnahmebehälter 2 eingeführt werden.

Die Probenentnahmeeinrichtung 8 weist zumindest ein hohles stabförmiges Element 16 auf. An dem freien Ende des stabförmigen Elements 16 ist das Entnahmeelement 12 angeordnet. Das hohle stabförmige Element 16 bildet im Inneren einen Kanal 18. Das stabförmige Element 16 und damit die Probenentnahmeeinrichtung 8 sind mit der Verschlusseinrichtung 6 verbunden.

Die Verschlusseinrichtung 6 weist eine Durchgangsöffnung 20 auf. Das Probenentnahmeelement und damit das stabförmige Element 16 sind derart mit der Verschlusseinrichtung 6 verbunden, dass der Kanal 18 in der Durchgangsöffnung 20 mündet oder in dieser angeordnet ist. Das stabförmige Element 18 kann somit in der Durchgangsöffnung 20 angeordnet sein und mit der Verschlusseinrichtung 6 verbunden sein oder am dem der Probenentnahmeeinrichtung 8 zugewandten Ende mit der Verschlusseinrichtung 6 verbunden sein, so dass der Kanal 18 in die Durchgangsöffnung 20 mündet.

In Fig. 2 ist dargestellt, wie die Probe, insbesondere eine Speichelprobe, entnommen werden kann. Dafür kann die Probenentnahmeeinrichtung 8 durch die Nase oder durch den Mund eingeführt werden und mit dem Entnahmeelement 12 kann die Probe entnommen werden.

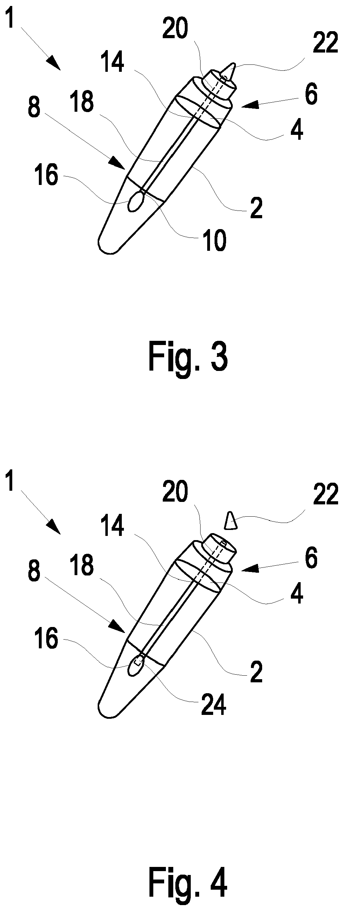

In Fig. 3 ist dargestellt, wie der Aufnahmebehälter 2 mit der Verschlusseinrichtung 6 verschlossen ist. Dabei schließt die Verschlusseinrichtung die Öffnung 4 ab. Mit einer Verschlusseinrichtung kann der Aufnahmebehälter 2 flüssigkeitsdicht und vorzugsweise luftdicht verschlossen sein. Die Länge der Probenentnahmeeinrichtung 8 ist derart gewählt, dass bei verschlossener Verschlusseinrichtung 6 die Probenentnahmeeinrichtung 8 nicht gegen den Boden des Aufnahmebehälters 2 stößt. Vorzugsweise ist die Probenentnahmeeinrichtung 8 jedoch so lang, dass, wenn eine Flüssigkeit in dem Aufnahmebehälter 2 angeordnet ist, das Entnahmeelement 12 in der Flüssigkeit eingetaucht ist.

Nachdem eine Probe mit dem Entnahmeelement 12 entnommen worden ist, kann die Probenentnahmeeinrichtung 8 in den Aufnahmebehälter 2 eingeführt werden, ohne dass Flüssigkeit in dem Aufnahmebehälter 2 angeordnet ist. In diesem Fall kann dann mittels der Verschlusseinrichtung 6 die Öffnung 4 verschlossen werden und die gesamte Vorrichtung sicher transportiert werden, ohne dass Kontaminationen auf die Probe kommen. In einem Labor oder Ähnlichem kann dann die Verschlusseinrichtung 4 wieder geöffnet werden und Flüssigkeit eingeführt werden. Die Probenentnahmeeinrichtung 8 kann dann wieder in den Aufnahmebehälter 2 eingeführt werden, so dass das Entnahmeelement 12 in die Flüssigkeit eintaucht.

Alternativ kann auch direkt Flüssigkeit in dem Aufnahmebehälter 2 angeordnet sein, so dass nach Entnahme der Probe die Probenentnahmeeinrichtung 8 in den Aufnahmebehälter 2 eingeführt wird und das Entnahmeelement 12 direkt in die Flüssigkeit eingetaucht wird. Auch dann kann die Öffnung 4 mittels der Verschlusseinrichtung 6 verschlossen werden.

Es ist ferner in Fig. 3 ein Abschlusselement 22 dargestellt, das die Durchgangsöffnung 20 des Verschlusselements 6 nach außen hin verschließt. Das Abschlusselement 22 ist trennbar mit dem Verschlusselement 6 verbunden. Solange das Abschlusselement 22 mit dem Verschlusselement 6 verbunden ist, kann keine Flüssigkeit durch den Kanal 18 und die Durchgangsöffnung 20 nach außen austreten.

In Fig. 4 ist die Vorrichtung gemäß Fig. 3 dargestellt, jedoch mit dem Unterschied, dass das Abschlusselement 22 von dem Verschlusselement 6 abgetrennt worden ist. Vorzugsweise kann das Abschlusselement 22 von dem Verschlusselement 6 durch Abknicken abgetrennt werden.

Das hohle stabförmige Element 16 kann an dem freien Ende 10 zumindest eine Öffnung aufweisen. Die Öffnung kann die Öffnung des in dem stabförmigen Element angeordneten Kanal bilden. Im vorliegenden Fall kann dies die Öffnung 24 sein, die in dem Entnahmeelement 12 angeordnet ist. Die Flüssigkeit, die durch das Entnahmeelement 12 durchtritt, kann dann durch den Kanal 18 zur der Durchgangsöffnung 22 geführt werden und dort bei abgetrenntem Abschlusselement 22 aus dem Aufnahmebehälter 2 austreten. Die Flüssigkeit kann aufgrund von Kapillarwirkung und/oder Druck und/oder Unterdruck zu der Durchgangsöffnung 22 geführt werden und dort bei abgetrenntem Abschlusselement 22 aus dem Aufnahmebehälter 2 austreten.

Dies ist beispielsweise in Fig. 4 dargestellt. Dort wird Flüssigkeit aus dem Aufnahmebehälter 2 ausgeschüttet in ein erstes Probenröhrchen A1. Dies kann dann zur weiteren Untersuchung weiterverwendet werden.

Die Öffnung in dem stabförmigen Element 16 kann auch in der umlaufenden Wand angeordnet sein und/oder kann auch in dem Bereich angeordnet sein, der an die Verschlusseinrichtung 6 angrenzt. Es kann auch sowohl in diesem Bereich zumindest eine Öffnung angeordnet sein und zusätzlich noch eine Öffnung im Bereich des Entnahmeelements 12.

## Patentansprüche

1. Vorrichtung (1) zur Aufnahme von Proben, insbesondere Speichelproben zum Nachweis von SARS-CoV-2 Virus im Speichel, mit
- einem Aufnahmebehälter (2) mit einer ersten Öffnung (4),
- einer Verschlusseinrichtung (6), die an die Öffnung (4) des Aufnahmebehälters (2) angepasst ist, so dass die Öffnung (4) des Aufnahmebehälters (2) mit der Verschlusseinrichtung (6) verschließbar ist,
- einer Probenentnahmeeinrichtung (8), die an einem ersten freien Ende (10) zumindest einen ersten Entnahmeelement (12) aufweist, mit dem die Probe entnehmbar ist, und wobei die Probenentnahmeeinrichtung (8) in den Aufnahmebehälter (2) einführbar ist,
**dadurch gekennzeichnet,**
**dass** die Probenentnahmeeinrichtung (8) an einem dem ersten freien Ende (10) gegenüberliegenden zweiten Ende (14) mit der Verschlusseinrichtung (6) verbunden ist, so dass die Probenentnahmeeinrichtung (8) mittels der Verschlusseinrichtung (6) in den Aufnahmebehälter (2) einführbar ist.

2. Vorrichtung nach Anspruch 1, **dadurch gekennzeichnet, dass** die Probenentnahmeeinrichtung (8) zumindest ein hohles stabförmiges Element (16) aufweist, an dessen freien Ende das Entnahmeelement (12) angeordnet ist, wobei das hohle stabförmige Element (16) im Inneren einen Kanal (18) bildet.

3. Vorrichtung nach Anspruch 1 oder 2, **dadurch gekennzeichnet, dass** die Verschlusseinrichtung (6) eine Durchgangsöffnung (20) aufweist.

4. Vorrichtung nach einem der Ansprüche 1 bis 3, **dadurch gekennzeichnet, dass** das stabförmige Element (16) derart mit der Verschlusseinrichtung (6) verbunden ist, dass der Kanal (18) des stabförmigen Elements (16) in der Durchgangsöffnung (20) mündet oder in dieser angeordnet ist.

5. Vorrichtung nach Anspruch 4, **dadurch gekennzeichnet, dass** die Durchgangsöffnung des Verschlusselements an der dem Probenentnahmeelement gegenüberliegenden Seite mittels eines Abschlusselements verschlossen, das mit dem Verschlusselement trennbar befestigt ist.

6. Vorrichtung nach Anspruch 5, **dadurch gekennzeichnet, dass** das Abschlusselement von dem Verschlusselement durch Abknicken trennbar ist.

7. Vorrichtung nach einem der Ansprüche 1 bis 6, **dadurch gekennzeichnet, dass** das hohle stabförmige Element an dem freien Ende zumindest eine Öffnung aufweist.

8. Vorrichtung nach Anspruch 7, **dadurch gekennzeichnet, dass** das Ende des in dem stabförmigen Element angeordneten Kanals die Öffnung am freien Ende bildet.

9. Vorrichtung nach Anspruch 7 oder 8, **dadurch gekennzeichnet, dass** die an dem freien Ende angeordnete Öffnung in der Wand des stabförmigen Elements angeordnet ist.

10. Verfahren zur Aufnahme von Proben,
- durch Bereitstellen eines Aufnahmebehälters (2) mit einer ersten Öffnung (4),
- Entnahme einer Probe mit zumindest einem an einem freien Ende einer Probenentnahmeeinrichtung (8) angeordneten Entnahmeelements (12),
**dadurch gekennzeichnet,**
**dass** die Probenentnahmeeinrichtung (8) einem dem ersten freien Ende (10) gegenüberliegenden zweiten Ende (14) mit einer Verschlusseinrichtung (6) verbunden ist und mittels der Verschlusseinrichtung (6) in den Aufnahmebehälter (2) eingeführt wird und der Aufnahmebehälter mit der Verschlusseinrichtung verschlossen wird.

11. Verfahren nach Anspruch 10, **dadurch gekennzeichnet, dass** die Flüssigkeit in dem Aufnahmebehälter eingeführt wird und das Entnahmeelement (12) der Probenentnahmeeinrichtung (8) beim Einführen in den Aufnahmebehälter in die Flüssigkeit eingetaucht wird.

12. Verfahren nach Anspruch 11, **dadurch gekennzeichnet, dass** die Flüssigkeit durch einen Kanal, der in einem stabförmigen Element der Probenentnahmeeinrichtung angeordnet ist, zu einer Durchgangsöffnung der Verschlusseinrichtung geführt werden kann.

13. Verfahren nach einem der Ansprüche 10 bis 12, **dadurch gekennzeichnet, dass** die Durchgangsöffnung mit einem Abschlusselement verschlossen ist und dass das Abschlusselement von der Verschlusseinrichtung abgetrennt werden kann.

14. Verfahren nach Anspruch 13, **dadurch gekennzeichnet, dass** die Flüssigkeit, die durch den Kanal, der in einem stabförmigen Element der Probenentnahmeeinrichtung angeordnet ist, zu der Durchgangsöffnung der Verschlusseinrichtung geführt wird, aus der Durchgangsöffnung ausgeschüttet werden kann, nachdem die Abschlusselement von der Verschlusseinrichtung abgetrennt worden ist.
